Europäisches Patentamt

(19) European Patent Office

Office européen des brevets

(11) Publication number: **0 205 598 B1**

# EUROPEAN PATENT SPECIFICATION

(45) Date of publication of patent specification: **29.01.92**   (51) Int. Cl.⁵: **C07C 2/52**, C07C 2/00

(21) Application number: **86900586.8**

(22) Date of filing: **30.12.85**

(86) International application number:
**PCT/US85/02593**

(87) International publication number:
**WO 86/04054 (17.07.86 86/17)**

(54) **NATURAL GAS CONVERSION PROCESS.**

(30) Priority: **31.12.84 US 688058**
**26.03.85 FR 8504454**
**29.07.85 FR 8511534**
**12.12.85 US 807976**

(43) Date of publication of application:
**30.12.86 Bulletin 86/52**

(45) Publication of the grant of the patent:
**29.01.92 Bulletin 92/05**

(84) Designated Contracting States:
**DE GB NL**

(56) References cited:
**FR-A- 1 323 474       US-A- 4 100 218**
**US-A- 4 350 835       US-A- 4 465 893**
**US-A- 4 507 517       US-A-41 995 33**
**US-A-44 436 47        US-A-44 503 10**

(73) Proprietor: **GONDOUIN, Oliver Michael**
**32 San Marino Drive**
**San Rafael, CA 94901(US)**

(72) Inventor: **GONDOUIN, Oliver Michael**
**32 San Marino Drive**
**San Rafael, CA 94901(US)**

(74) Representative: **Colas des Francs, Jean**
**Institut Français du Pétrole 4, Avenue de**
**Bois Préau**
**F-92502 Rueil-Malmaison(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention is concerned with an improved process for converting natural gas into a low-vapor pressure liquid hydrocarbon mixture, which for instance can be more easily transported from a remote oil or gas field to market. More particularly, this invention deals with a novel processing scheme to convert light gases (methane to butanes) into aromatics, C5 + hydrocarbons and a hydrogen-rich by-product gas. This process, if applied in a petroleum refinery, also allows to increase the yield of higher value liquid products (BTX) at the expense of less valuable gas products.

Description of Prior Art

Conversion of the C1-C4 fractions of natural gas into higher molecular weight components is commonly achieved by first fractionating the feed into essentially pure components, such as ethane or propane and then converting each fraction individually into heavier, more valuable components such as olefins or aromatics, using either non-catalytic thermal processes (e.g. steam pyrolysis or thermal cracking for the conversion of ethane into ethylene), or catalytic dehydrogenation processes or a combination of both types of processes in which an essentially pure lighter feed component, ethane for example, is first converted into ethylene by steam cracking at high temperature, and the ethylene is separated and subsequently converted to aromatics catalytically at moderate temperature in a second step. The catalysts used are composed primarily of silica ($SiO_2$) in the form of silicates or alumino-silicates. It is claimed that the acidic nature of the silica-based catalysts (Bronsted acid sites) or the cage-like structure of the catalyst crystal (such as ZSM-5 zeolite) are determining factors in these conversion processes. Some of these processes are oxidative in nature and result in the formation of oxidation products, such as water, which have no fuel value and must be separated from the hydrocarbon product. Still, other processes use "super acids" as catalysts, including various oxidative reagents (fluorine, chlorine, sulfates). These processes also result into oxidation products which must be separated out for disposal or for reclaiming.

Some of the known catalytic processes for the conversion of lighter components C1, C2, of natural gas, mostly produce light olefins and LPG components which must then be separated out and converted to heavier components in a second step, using different catalysts.

The early commercial processes (Fischer-Tropsch) available to produce hydrocarbon liquids from gases did not use natural gas as a feed stock but various mixtures of CO and $H_2$. These can be derived from natural gas by various oxidative processes, so that the conversion of natural gas into hydrocarbon liquids requires additional pre-processing and the use of several different catalysts. These processes based on syngas (CO + $2H_2$) also proceed through many intermediate steps in which oxygenated products (methanol for instance) are formed and must be separated, purified and de-oxygenated into other intermediate chemical specie before reaching the final goal of a marketable liquid hydrocarbon mixture. The catalysts used in these processes generally contain large amounts of silica. Thermal pyrolysis of methane in an electric arc furnace has been used to produce acetylene, which is a much more stable component at very high temperatures than aromatics or naphtha, in the absence of any catalyst. Although acetylene is an important raw material for organic synthesis, it is not easily transportable, and cannot be marketed together with crude oil or condensate as a liquid mixture.

U.K. Patent Application GB 2,148,935A published June 6, 1985 by L. DeVries et al. [and assigned to Chevron Research Co. of San Francisco, CA. (U.S.A.)] discloses a process for the catalytic production of higher molecular weight hydrocarbons. Very high reaction temperatures greater than 1000° C and preferably greater than 1100° C are disclosed.

Furthermore, this conversion process, which is largely endothermic, may make use of surplus electrical power, also available in summer, to provide an inexpensive source of heat for the pre-heating furnace and for producing the hydrogen plasma required by this process.

Storage of the hydrocarbon liquids resulting from the conversion is rather inexpensive, so that the LNG conversion process, when using electricity as a source of heat, may be considered as a way of indirectly storing surplus electrical power derived in the summer from nuclear plants or from hydroelectric plants.

SUMMARY OF THE INVENTION

Case of a Natural Gas Rich in Ethane and Heavier Hydrocarbons

The present invention (as summarized on page 38) combines the use of a single catalyst over a wide range of temperatures to convert successively, (1) a mixture of the heavier components of the natural gas (principally the available propane and butanes with a portion of the available ethane) at moderate temperatures and relatively long residence times, in a catalytic bed reactor, and (2) a mixture of light gas components C1, C2, and $H_2$ at high temperature and very short residence times in a cyclone reactor. The conversion order may also be reversed. The first step of the process is therefore to split the feed gas into a rich gas stream (C2, C3, C4), and a lean gas stream (C1, C2); this is accomplished in a feed splitter unit of conventional design. The catalyst used for this dual conversion process is handled as a fluidized or moving bed which, for the rich gas conversion, may be subject either to an upward flow in a reactor similar to a fluid catalytic cracking (FCC) reactor, or to a gravity-assisted flow in a moving bed reactor similar to CCR or TCC reactors. For the conversion of lean gas (C1, C2), the catalyst flowing respectively to the top or bottom of the catalytic bed reactor in these two cases, when the rich gas conversion occurs first, reaches an overflow standpipe of conventional design leading into the cyclone reactor, through an inlet located at the periphery of the cylindrical cavity of the cyclone. The catalyst particles falling into the cyclone reactor meet the tangential flow of the pre-heated lean gas feed into the cyclone. This gas flow carries the catalyst in a downward spiral motion against the conical surface of the cyclone reactor, and from there it is led into a disengagement cyclone.

The reaction products are separated from the spent catalyst in the disengagement cyclone and in a conventional steam stripper. In the cyclone reactor a cold enriched hydrogen stream is fed through an axial insulated pipe into the reactor, where it is subjected to intense heat by electricity or other known means (for instance a high frequency electromagnetic field combined with a starter electric arc). As a result, the hydrogen is dissociated into an ionized plasma confined to a central region located along the vertical axis of the cyclone reactor.

The lean gas feed stream to the cyclone reactor, is pre-heated in a conventional furnace to a temperature of 700° C to 1000° C (but not exceeding the feed's incipient pyrolysis temperature). It is then injected tangentially into the cylindrical cavity of the cyclone reactor.

The catalyst fed into the cyclone reactor is at a temperature significantly lower, less than 600° C. Despite the high temperature of the feed gas, the peripheral wall temperature of the cyclone remains well below 900° C.

The temperature of the catalyst itself does not exceed 900° C when it comes out of the cyclone part of the reactor, but the refractory properties of the oxide components of the catalyst allow it to withstand the thermal shock resulting from local short term exposure to the very high temperature central region of the reactor without degradation of the crystalline structure of the catalyst. Further cooling of the catalyst and of the reaction products occurs in the heat recovery section of the reactor below the conical cavity of the cyclone. The rich gas stream is the cooling medium.

The chemical composition of the dehydrogenation catalyst is characterized by the following formula:

$$x \, MO_2, \, y \, M'M'' \, O_3, \, z \, M'' \, ' \, O_2$$

where x, y and z are mole fractions ranging from 0 to 98 percent, and x is greater than y, which is greater than z,

M      is an actinide divalent metal, such as thorium,

M'      is a trivalent metal such as gallium,

M''     is a lanthanide trivalent metal, such as lanthanum,

M'' '   is a divalent metal such as zirconium or hafnium.

In one embodiment, the catalyst comprises preferably at least one metal oxide selected from the oxides of metals of Groups III A, III B, and/or IV B, and mixtures thereof as is defined in the Hodgman Handbook (a standard reference). In a preferred embodiment, the catalyst comprises between about 0.5 to 80 percent of Group III A, 0 to 80 percent of Group III B and 0 to 80 percent of Group IV B as the metal oxides. A preferred metal oxide of Group III A is gallium oxide. An additional preferred composition comprises gallium oxide, an oxide of a lanthanide metal and an oxide of an actinide metal. A further preferred composition comprises between about 0.5 to 30 percent of gallium oxide, 1 to 50 percent of thorium oxide, 0.5 to 30 percent of a lanthanide oxide and 0 to 30 percent of a Group IV B metal oxide. The Group IV B metal oxide is preferably zirconium oxide in an amount of between about 0.5 to 20 percent. The catalyst can tolerate small amounts of hydrogen sulfide and of sulfur components without being poisoned. It can also tolerate the presence in the natural gas feed of significant percentages of carbon dioxide, especially if the carbon dioxide fraction is in majority directed to the rich gas stream from the feed splitter unit.

The injection of relatively cold catalyst into the cyclone reactor quenches the dehydrogenation reactions and provides an effective screen against heat radiation from the central plasma region towards the cylindrical and conical walls of the cyclone reactor. The high velocity spiral flow of catalyst particles suspended in the gas phase facilitates the transfer of heat towards the outside through the reactor wall.

Heat radiation towards the top of the reactor is reduced by the use of a heat shield made of known refractory materials (ceramics, carbides, tungsten foil, graphite, etc.). Further cooling of the top wall of the reactor is achieved by the injection of a secondary cold hydrogen-rich stream into inlets located at the periphery of the top wall. The cooling gas flows above the heat shield in spiral or helicoidal channels along guiding vanes below the top wall of the reactor, and exits into the cylindrical cavity near the center of the reactor, through openings in the heat shield. The spiraling secondary hydrogen stream, heated by contact with the back face of the heat shield then flows downward in a swirling motion around the plasma jet, and exits through the reactor outlet either unseparated from the spent catalyst or mixed with the separated gaseous phase containing the reaction products. Along this path many molecules of the secondary hydrogen stream become ionized by the plasma. The hydrogen ions collide with gas molecules contained in the tangential inlet feed stream which, by design, rotates in the opposite direction of the secondary hydrogen stream. The increased number of collisions of relatively low energy hydrogen ions with hydrocarbon molecules and with free radicals enhances the yield of the dehydrogenation reactions.

The usual time from the formation of the hydrogen plasma to contact with the reactants and catalyst is very short. Normally, the time is instantaneous (nanoseconds) up to a maximum of about 500 milliseconds. More preferably, the time is between about 100 and 500 milliseconds.

Although the catalyst inhibits the formulation of carbons, it eventually becomes loaded with coke, with a corresponding reduction in effectiveness. Regeneration of the catalyst is achieved in a conventional manner by burning the coke in a fluidized or moving bed regenerator at temperatures not exceeding $600^\circ$C. The regenerated catalyst is maintained in an oxygen-free atmosphere until it is recycled into the catalytic bed reactor for conversion of additional C3, C4 from the rich gas feed.

The spent catalyst coming out of the cyclone reactor and separated from the gaseous effluent of the reactor in the disengagement cyclone contains not only coke, but also some of the heavy hydrocarbon components generated by the dehydrogenation reactions. These heavy hydrocarbons are stripped off the catalyst with steam in a conventional fluidized bed stripper, preferably located directly below the disengagement cyclone, so that the stripped hydrocarbons and steam may be entrained by the gaseous effluent from the disengagement cyclone. Such an arrangement is well known from those skilled in the art.

The hydrogen required for the primary and secondary hydrogen streams fed into the cyclone reactor is obtained by separation from the gaseous effluents streams of both the catalytic bed reactor and the cyclone reactor. These two effluents are separated by conventional means into four streams:
- a hydrogen-rich stream
- a lean gas ($H_2$, C1, C2) recycle stream
- a rich gas (C2, C3, C4) recycle stream
- a product (C5 + ) stream

For economies of scale, the separation unit is preferably the same for both effluent streams and may include for instance:
- a gas chiller to condense the C2 + fraction,
- a low temperature separator (LTS) separating $H_2$, C1, C2 vapor phase,
- a hydrogen extraction unit which may operate by pressure swing adsorption or by membrane separation, cryogenic separation, or any other method known to those skilled in the art.
- a fractionation column to separate the C5 + product from the C2, C3, C4 fraction of the LTS condensed liquid.
- compression means and auxiliary facilities as required for such a conventional process which is well known of those skilled in the art. The separation and feed splitter may share facilities.

The transfer of catalyst from the regenerator to the catalytic bed reactor is accomplished by conventional means, (transfer lines, riser reactor, lift pot, lift lines, etc.) well known of those skilled in the art.

In a preferred embodiment of the invention, the cyclone reactor is located within the catalytic bed reactor so that the heat flux through the cyclone reactor wall contributes to heating the catalytic bed reactor. Another source of heat for the catalytic bed reactor is provided by the hot regenerated catalyst resulting from the coke combustion.

## BRIEF DESCRIPTION OF THE DRAWINGS

Fig. 1 is a schematic block diagram of the natural gas conversion process, showing the catalyst circulation and the flow of feed, products and recycle streams when rich gas conversion occurs first.

Fig. 2 is a vertical cross section of the cyclone reactor showing the respective disposition of the inlets for the lean gas feed, the catalyst, the primary hydrogen stream, the secondary hydrogen. It also shows the

EP 0 205 598 B1

respective paths of the secondary hydrogen and of a tangential stream of lean gas feed and catalyst. The high frequency coil used to produce the ionized hydrogen plasma is shown schematically. The heat recovery section is also shown schematically.

Fig. 3 is a horizontal cross section AA of the top of the cyclone reactor, showing the spiral channels used to impart a swirling motion to the secondary hydrogen stream in the opposite direction of that of the lean gas feed and catalyst stream.

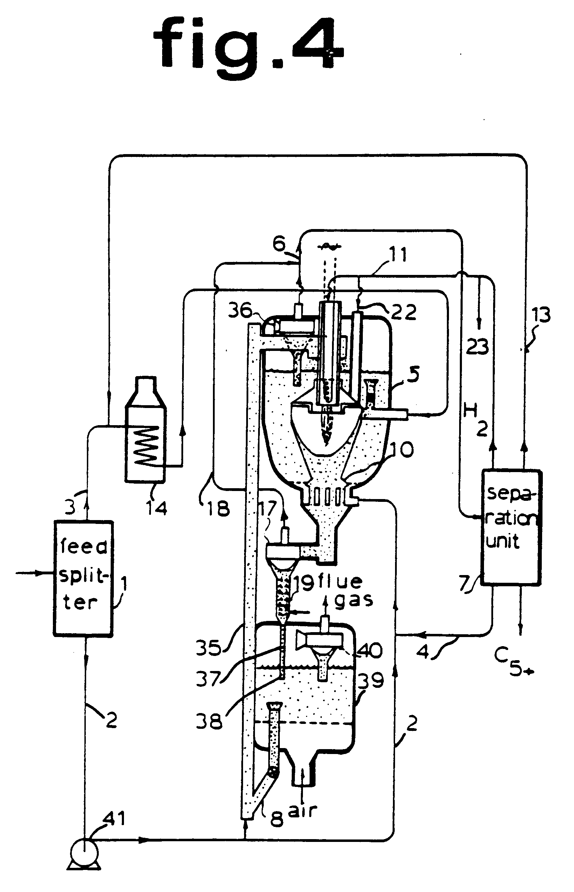

In the embodiment shown on the flow diagram of Fig. 4, the cyclone reactor is located within a fluidized bed reactor. The transfer of regenerated catalyst back to the fluidized bed reactor is obtained by means of a riser reactor. The feed gas splitting unit and the reactor effluent gas separation unit, both of conventional design, are not detailed.

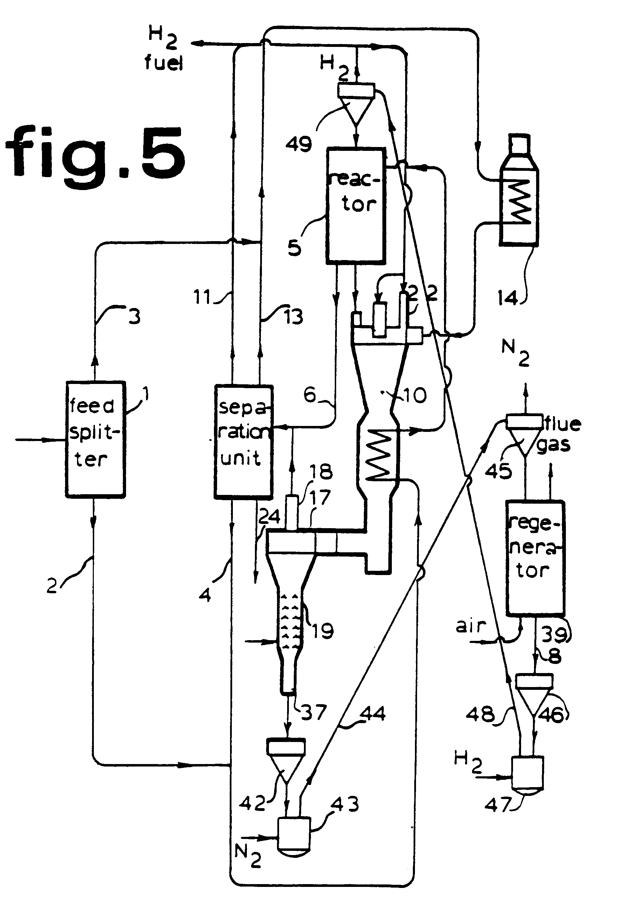

In the embodiment shown on the flow diagram of Fig. 5, the catalytic bed reactor is a moving bed reactor of conventional design, and is not detailed. The transfer of the spent catalyst into the regenerator and of the regenerated catalyst back into the moving bed reactor is obtained by means of lift pots and lift lines of conventional design.

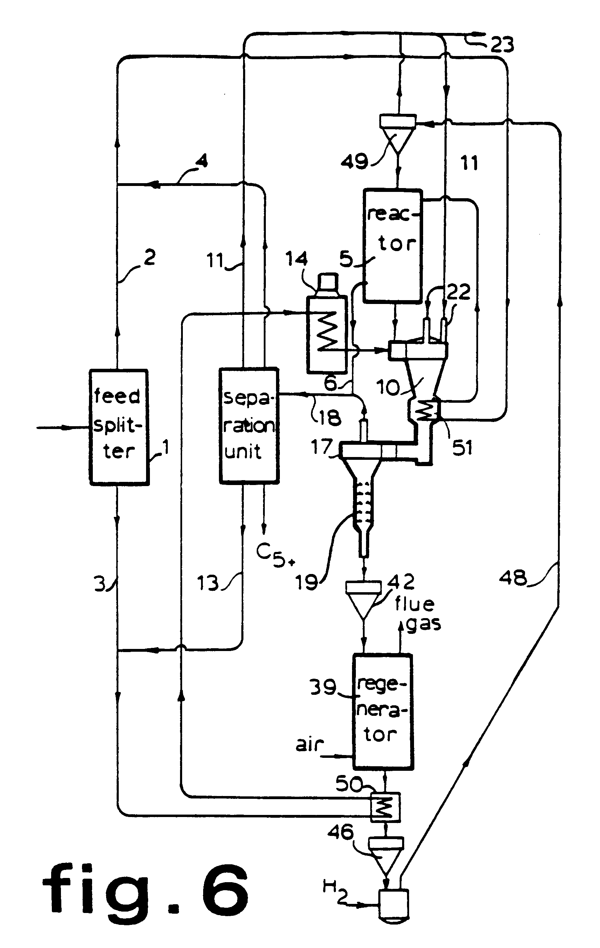

Fig. 6 is a flow diagram of another embodiment of the process in which the catalyst flows by gravity from a moving bed reactor at the top into the cyclone reactor below, and descending from there to the disengagement cyclone, the steam stripper, and finally the moving bed regenerator. The regenerated catalyst is then collected in a lift pot at the bottom and transported upwards by lift gas to a hopper above the moving bed reactor.

The moving bed reactor, moving bed regenerator, and catalyst transport system, all of the conventional design, are not detailed.

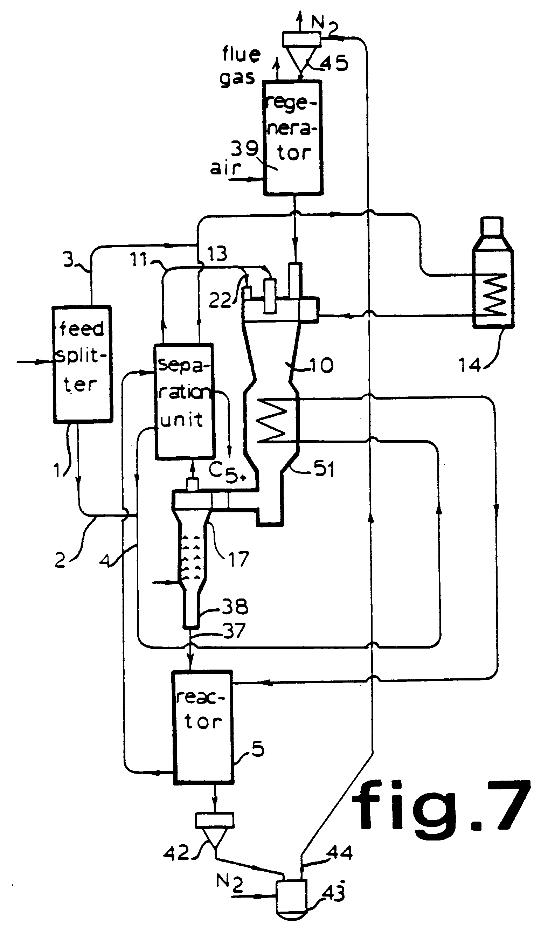

A case in which the lean gas conversion occurs first, followed by rich gas conversion in a moving bed reactor is shown on Fig. 7. In this variant of the process, the regenerated catalyst is used for lean gas conversion and the stripped catalyst is used for rich gas conversion.

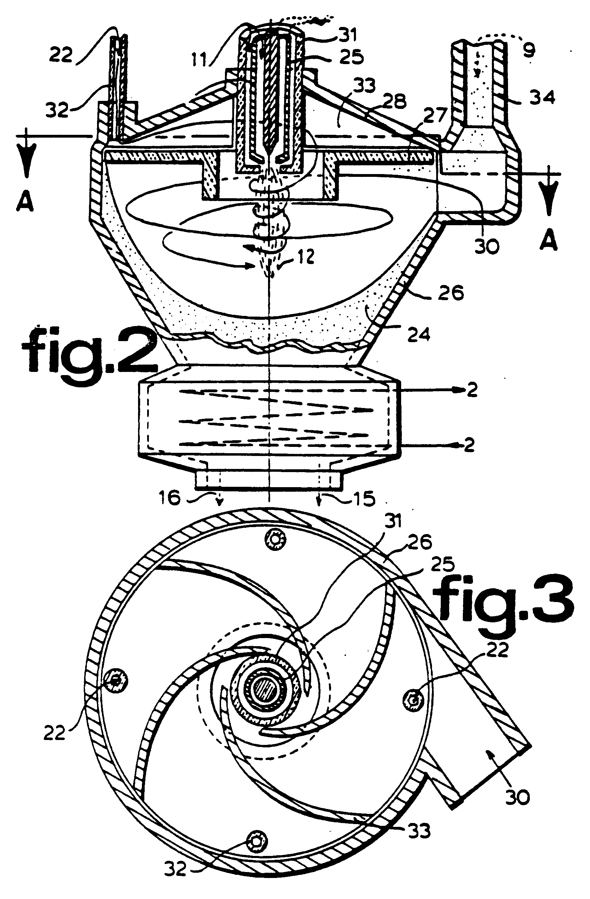

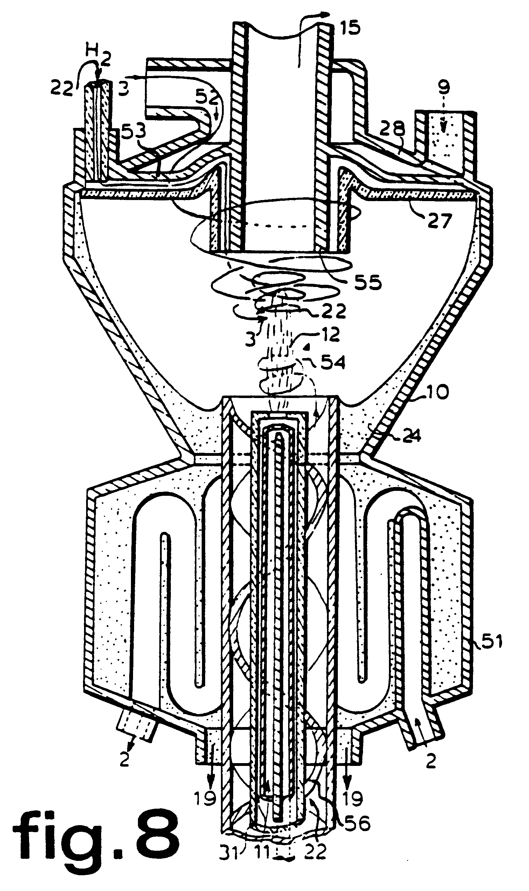

Fig. 8 shows another embodiment wherein the functions of the cyclone reactor and of the disengagement cyclone are combined in a single vessel. The hydrogen plasma jet in this case flows upwards instead of downwards, as in Fig. 2 and Fig. 4. Fig. 8 shows a vertical cross-section of the vessel and internals which combine the functions of cyclone reactor, disengagement cyclone and heat recovery section.

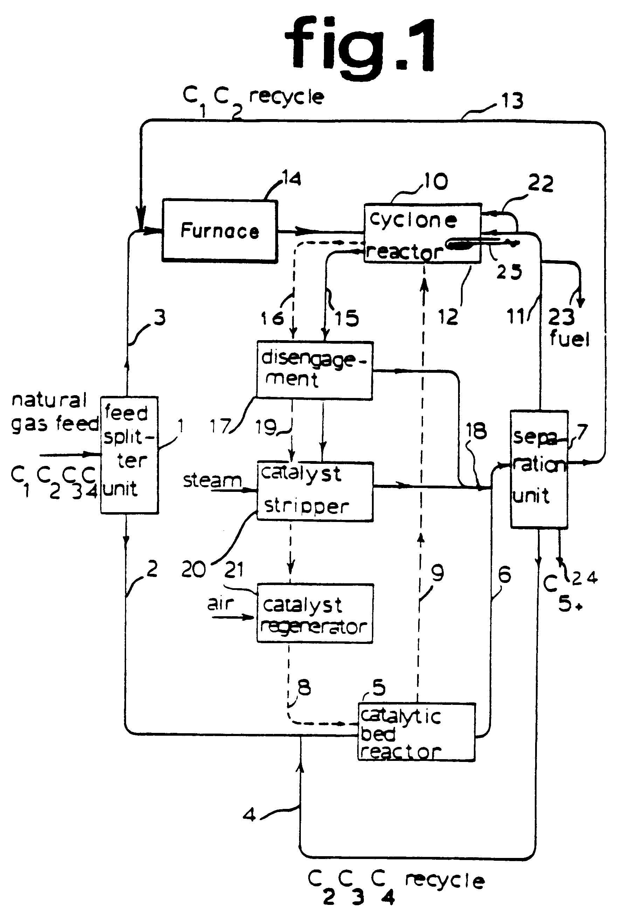

Fig . 9 is an other kind of a schematic block diagrm, derived from Fig. 1.

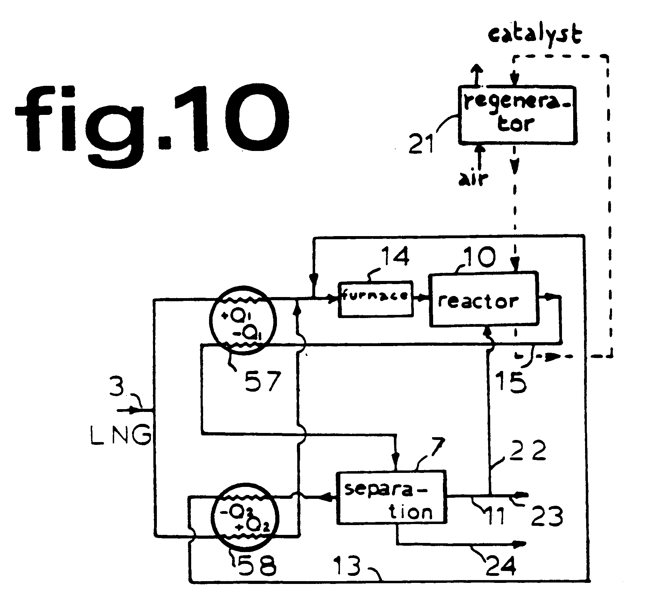

Fig. 10 is a schematic block diagram of the LNG conversion process, including the flow of catalyst and its regeneration in an oxygenated stream. The heat produced in the regenerator may be recovered from the stack gases to provide re-boiler heat for the separation unit.

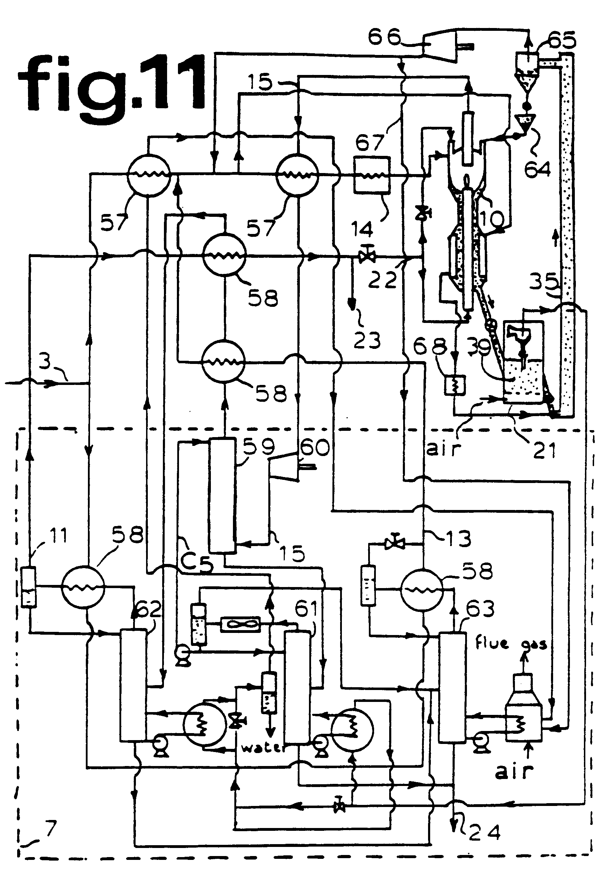

Fig. 11 is a process flow diagram showing an example of application in which a separation unit based on cryogenic fractionation is detailed.

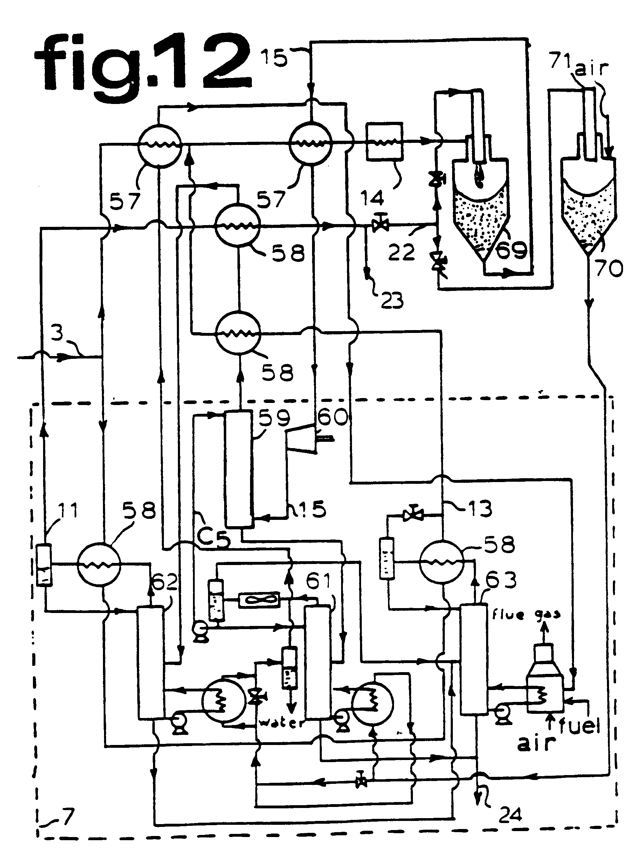

Fig. 12 is a process flow diagram showing an example of application in which the cyclone reactor is replaced by a fixed bed reactor, with a longer residence time and higher operating temperature.

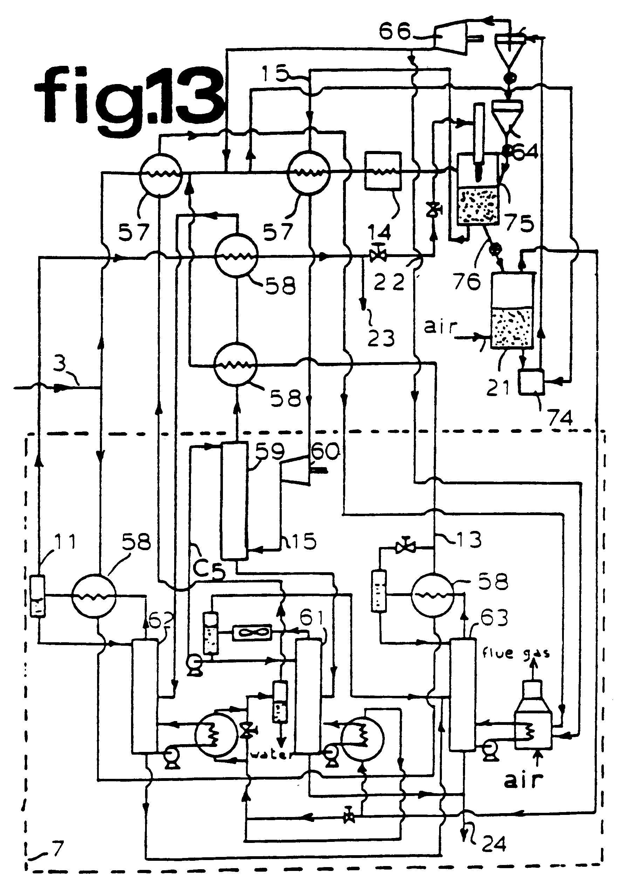

Fig. 13 is a process flow diagram showing an example of application in which both the reactor and the regenerator are of the moving bed type.

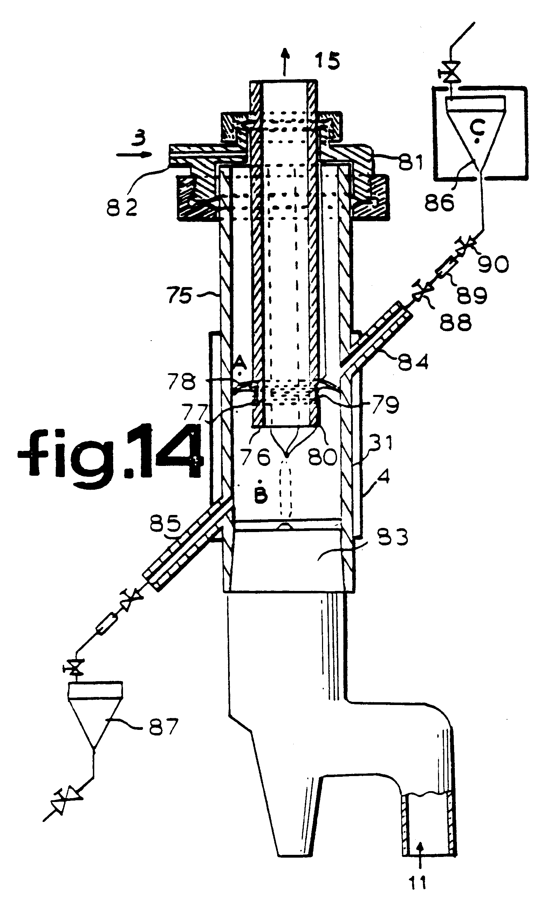

Fig. 14 is a vertical cross-section of a laboratory apparatus used to demonstrate the effects of the catalyst and of the hydrogen plasma on the conversion of methane-rich feeds in a cyclone reactor, and of propane-rich feeds in a fluidized bed reactor.

## DETAILED DESCRIPTION OF THE INVENTION

Process Description:

Referring to Fig. 1, the following successive steps of the conversion process when rich gas conversion occurs first are as follows:

(1) In the splitter unit (1) of conventional design, the natural gas feed is split into a rich gas feed (2), and a lean gas feed (3).

(2) The rich gas feed (2), together with the C2, C3, C4 recycle stream (4) from the separation unit is fed into the catalytic bed reactor (5).

(3) The gaseous effluent (6) from the catalytic bed reactor is fed into the separation unit (7) of conventional design.

(4) The regenerated catalyst (8) enters the catalytic bed reactor and exits from it (9), to enter the cyclone reactor (10).

(5) The hydrogen stream (11) obtained in the separation unit (7) is injected into the cyclone reactor (10) to produce the ionized hydrogen plasma (12).

5

(6) The lean gas stream (3) together with the C1, C2 recycle stream (13) obtained in the separation unit is heated in the furnace (14) prior to injection into the cyclone reactor (10).

(7) The gaseous effluent (15) from the cyclone reactor, together with the spent catalyst (16) enter the disengagement cyclone (17).

(8) The catalyst effluent (19) from the disengagement cyclone (17) is fed to the catalyst stripper (20) and from there to the catalyst regenerator (21).

(9) A portion of the hydrogen stream (11) is used as a secondary hydrogen cooling medium (22) and is fed to the cyclone reactor.

(10) The remainder (23) of the hydrogen generated in the separation unit is diverted for use as process fuel or for other applications.

(11) The liquid hydrocarbon product (24) obtained from the separation unit is recovered for sale.

(12) The block diagram of Fig. 1 shows a coil (25) indicating that the primary hydrogen stream is subjected to a high frequency electromagnetic field in order to produce the ionized plasma.

The same designations and numbers will be used in the other Figures in which these elements 1 to 25 also appear with the same meaning as in Fig. 1, if not repeated in the description.

Preparation of the Catalyst

The catalyst is prepared by co-precipitation of insoluble salts of the metals M, M', M'', M'' ' and of an acid which is easily decomposed by heat. Typical examples of such salts are benzoates, oxalates, carbonates, etc. The precipitate may be formed by the reaction of a water and alcohol solution of heat unstable acids such as benzoic, oxalic, or carbonic acids with an aqueous or alcohol solution of soluble salts of the said metals. Typical examples of such soluble salts are nitrates and acetates. In a specific example, a mixed solution of thorium, gallium, lanthanum and zirconium salts (nitrates for instance) is prepared and buffered by the addition of an organic base (diethanolamine for instance) at a temperature not exceeding $80°C$. Precipitation may also be obtained by slowly adding solutions in water and alcohol of salts from said acids in which the cations are alkali metals or ammonium. The precipitate is separated by filtration or centrifugation, washed with distilled water, and the wet paste is charged into an autoclave where it is maintained at a controlled temperature not exceeding $180°C$ for a week. The resulting mixed crystals are then air dried in an oven at $80°C$ and compacted into pellets. These are subsequently incinerated in an autoclave in successively increasing temperature steps. The dissociation of the heat - sensitive crystals into oxides is monitored by following the increase in pressure in the autoclave. At the end of each temperature step, the gaseous products of thermal dissociation are displaced with an inert gas such as steam, or nitrogen. A final step of the heat treatment of the catalyst pellets is their sintering in an inert atmosphere at a temperature not exceeding $1000°C$. The resulting mixed oxides pellets may then be used directly in a moving bed reactor or pulverized to a prescribed mesh size for use in a fluid bed reactor. The pulverized catalyst may also be mixed with an organic or inorganic binder, compressed or extruded into pellets and sintered at high temperature according to procedures well known to those skilled in the art.

The structure of the catalyst crystals is that of an irregular cubic system including many dislocations. This is attributed to the fact that below $900°C$, pure zirconia ($ZrO_2$) crystallizes in the monoclinic system whereas pure thoria ($ThO_2$) crystals are cubic, and gallium-lanthanum oxide ($GaLaO_3$) forms orthorombie (perovskite type) crystals. The rich gas conversion may be explained by the following successive reactions, overall largely endothermic:

$$C_3H_8 \rightarrow H^\bullet + C_3H_7^\bullet \qquad (1)$$

$$C_3H_7^\bullet + C_3H_8 \rightarrow H_2 + 2\, C_3H_6^\bullet + H^\bullet \qquad (2)$$

$$2\, C_3H_6^\bullet \rightarrow C_6H_6 + 3\, H_2 \qquad (3)$$

in which reaction (1) is rate limiting. Excited states are dotted. The lean gas (C1, C2) conversion is initiated through a different reaction, namely:

$$H_2 \rightarrow 2\, H^\bullet \qquad (4)$$

This reaction occurs in the plasma derived from a primary hydrogen-rich stream and generated by conventional means (e.g., electromagnetic) within the cyclone reactor. The conversion of C1, C2 is believed to proceed by the following reactions:

$$CH_4 + H^\bullet \rightarrow CH_3{}^\bullet + H_2 \quad (5)$$

$$CH_3{}^\bullet + CH_4 \rightarrow C_2H_6{}^\bullet + H^\bullet \quad (6)$$

$$C_2H_6{}^\bullet + H^\bullet \rightarrow C_2H_5{}^\bullet + H_2 \quad (7)$$

$$C_2H_5{}^\bullet + H^\bullet \rightarrow C_2H_4 + H_2 \quad (8)$$

$$3 C_2H_4 \rightarrow C_6H_6 + 3 H_2 \quad (9)$$

At high temperature, those reactions (5) to (9) are believed to proceed within the gas phase as well as on the catalyst surface. The dehydrogenation, especially in this gas phase, may continue further until final degredation into carbon and hydrogen, unless exposure of the desirable reaction products (C5 +) to the high temperature of the hydrogen plasma remains very short. This is achieved by quenching the intermediate dehydrogenation reactions (5 to 9) with the relatively cold (600°C) catalyst and by limiting the contact time of the gas phase in the high temperature region of the reactor. Further quenching occurs in the heat recovery section of the reactor. The liquefied natural gas conversion proceeds through the same reactions as that of the lean gas fraction of a natural gas but requires a slightly higher temperature.

Cyclone Reactor

Fig. 2 illustrates the concept of the cyclone reactor used to quench the dehydrogenation reactions and to protect the reactor walls (26, 28) from thermal radiation emitted by the plasma jet (12). The heat shield (27) made of refractory material protects the top wall (28). The catalyst settling on the cylindro-conical wall (26), and the catalyst in the dispersed phase (30) of the vortex, shield this wall from radiated heat. The primary hydrogen cold stream (11) introduced into the cyclone reactor by means of the insulated pipe (31) is ionized by the high frequency electromagnetic field produced by the cater cooled coil (25). The secondary hydrogen stream (22) is introduced into the cyclone reactor by means of the insulated pipe (32), and is guided along a spiral path by the guide vanes (33), also shown on cross-section on Fig. 3. The catalyst (9) is fed into the cyclone reactor by a standpipe (34) shown on both Fig. 2 and Fig. 3.

Embodiments of the invention

A preferred embodiment of the invention is shown on Fig. 4. The cyclone reactor (10) is located within a fluidized bed reactor (5) which is fed by a riser reactor (35) in which the regenerated catalyst (8) is entrained by the rich gas feed (2) and recycled rich gas (4).

The fluidized bed reactor (5) is similar in concept to that of a FCC reactor. It includes a cyclone (36) which provides the gas phase effluent (6) to the separation unit (7).

Also shown on Fig. 4 is the furnace (14) which pre-heats the lean gas feed prior to injection into the cyclone reactor (10). The gas-solid effluent stream from the cyclone reactor is sent to a disengagement cyclone (17) from which the spent catalyst (19) is fed directly into a steam stripper. The overhead gas effluent (18) from the disengagement cyclone contains all the gaseous reaction products including those stripped from the catalyst, plus unreacted feed and steam. The stripped catalyst (37) is fed by a dipleg (38) directly into the regenerator (39) of conventional design, which includes at least one stage of cyclone (40) to separate the flue gas from the regenerated catalyst (8). A pump (41) or compressor, if the rich gas feed is in vapor phase, provides the energy for the circulation of the catalyst. The lean gas phase fed into the furnace is also recompressed in the separation unit.

Another embodiment of the invention is shown on Fig. 5 in which the catalytic bed reactor (5) is a moving bed reactor of conventional design. The cyclone reactor is located directly below the moving bed reactor. All other elements of the process are the same as in Fig. 4, except that the regenerator (39) is also of the moving bed design, instead of being a fluidized bed regenerator as in Fig. 4.

The spent catalyst (37) is collected in a hopper (42) and transferred to a lift pot (43) and from there through a lift line (44) to a feed hopper (45) above the regenerator. Conversely, the regenerated catalyst (8) is collected in a hopper (46) and transferred to a lift pot (47), and from there to a lift line (48) leading to a feed hopper (49) above the moving bed reactor (5). This catalyst transport system, applicable to moving bed catalysts only is familiar to those skilled in the art.

The lift line (48), hopper (46) and lift pot (47) may be omitted in the embodiment of Fig. 5 when the moving bed regenerator (39) is located directly above the moving bed reactor (5) instead of being side by side as shown on Fig. 5.

Still another embodiment of the invention is shown on Fig. 6 in which the spent catalyst (19) flows by gravity directly from the stripper into the feed hopper (42) of the moving bed regenerator (39). On the flow diagram of Fig. 6, the regenerated catalyst exchanges its sensible heat with the lean gas stream in a waste heat recovery exchanger (50) before falling into the hopper (46). It is then lifted to the feed hopper (49) at the top of the moving bed reactor (5) by means of the lift line (48). As in the other two embodiments, the rich gas stream is pre-heated in the heat recovery section (51) of the cyclone reactor (10) prior to being fed to the catalytic bed reactor, in this case a moving bed reactor (5). In a variant of this embodiment, the moving bed regenerator may be located directly above the moving bed reactor (5) and the lift pot (43) and hopper (42) located downstream from the stripper, as in Fig. 5 are used in conjunction with a lift line (44) to feed the spent catalyst to the moving bed regenerator (39). The cooling fluids respectively in the heat recovery exchanger (50) and in the heat recovery section (51) of the cyclone reactor may also be interchanged.

In all previous embodiments the rich gas conversion occurs first. In the embodiment shown on Fig. 7, the lean gas conversion is obtained with freshly regenerated catalyst and the rich gas conversion is achieved in a moving bed reactor using the stripped catalyst resulting from the lean gas conversion in the cyclone reactor (10) and subsequent disengagement and stripping of the catalyst. The moving bed regenerator is located at the top, with the catalyst flowing by gravity from the regenerator (39) to the cyclone reactor (10) and from there into the disengagement cyclone (17) and steam stripper from which the stripped catalyst (37) is fed by a standpipe (38) into the moving bed reactor (5) in which conversion of the pre-heated rich gas takes place. The spent catalyst is then transferred to the feed hopper (45) of the moving bed regenerator (39) by means of a lift line (44) fed by the lift pot (43) and spent catalyst hopper (42). The lift gas is preferably an inert gas such as nitrogen. Pre-heating of the lean gas stream in the furnace (14) and of the rich gas stream in the heat recovery section (51) of the cyclone reactor (10) is obtained as in the previous embodiments. The feed splitter and the separation unit, both of conventional design also have the same functions as before.

In another embodiment of the invention shown on Fig. 8, the functions of the cyclone reactor and those of the disengagement cyclone are combined. The lean gas stream (3) is fed through an axial inlet (52) at the top and is guided into a rotational path by means of stationary helicoidal blades (53). The heat shield (27) is located immediately below the helicoidal blades (53). It forms an enclosed channel with each blade, through which a secondary hydrogen stream (22) flows from the inlet at the periphery towards the center in a helicoidal path. The swirling hydrogen streams then flow downward in a central vortex around the gas outlet of the cyclone reactor and into the cylindrical cavity around the central plasma jet (12).

The secondary cooling hydrogen downward vortices then collide with another secondary hydrogen upward vortex (54) surrounding the plasma jet and rotating in the opposing direction. All the hydrogen gas streams (11) (22) (54) then follow an upward helical path towards the axial gas outlet (55) of the cyclone at the top, together with the gas phase (15) separated from the spent catalyst (19). The spent catalyst (19) flows in a dense phase at the bottom into the heat recovery section (51) of the cyclone reactor, and from there into the catalyst stripper (20)(of Figure 1). Piping for the primary hydrogen stream and electrical power connections for the generation of the hydrogen plasma pass through the heat recovery section (51) in a hydrogen-cooled insulated pipe (31) along the axis at the lower part of the reactor. The secondary hydrogen cooling stream flows through an annular space concentric with the primary hydrogen pipe (31).

An helicoidal guide vane (56) within this annular space imparts to the secondary hydrogen stream a spiraling motion in the direction opposite to that of the lean gas stream (3). This disposition of the cyclone reactor shown on Fig. 8 may be substituted for that of Fig. 2 in each of the process diagrams shown on Fig. 1, 4, 5, 6, and 7.

When the disposition shown on Fig. 8 is used in the flow diagram of Fig. 7, the catalyst stripper may advantageously be omitted. This is possible because the faster disengagement of the lean gas conversion products from the catalyst results in smaller amounts of high molecular weight hydrocarbons saturating the catalyst. It was found however that the lean gas conversion yield increased with the presence of light hydrocarbons (ethane to benzene) within the catalyst pores prior to the conversion reaction. This may be obtained in the flow diagram of Fig. 7 by feeding a small amount of rich gas in the standpipe (34)[Figures 2 and 3] delivering fresh catalyst into the cyclone reactor (10). This optional disposition is not shown on Fig. 7, but may be implemented by those skilled in the art, using conventional techniques.

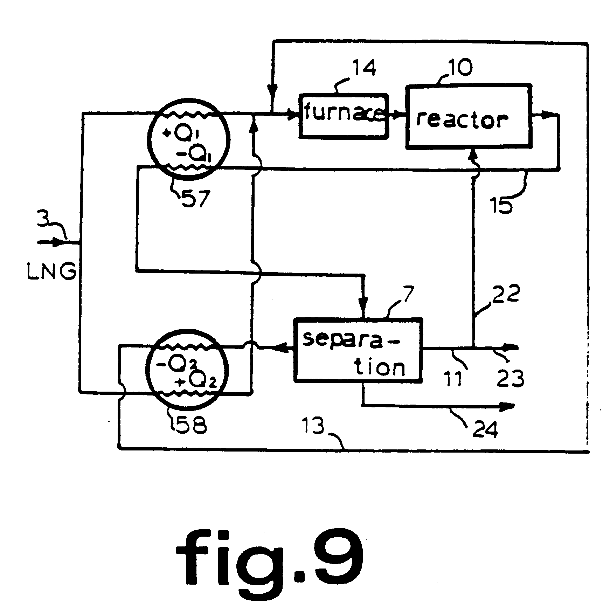

Fig. 9 to Fig. 14 relate to the conversion of liquified natural gas (LNG) which is similar to lean gas conversion. This is apparent when comparing the processes shown on Fig. 9 and Fig. 10 with that of Fig. 1.

Fig. 11 to Fig. 13 are embodiments of the invention, applicable to the case of an LNG feed.

8

Fig. 11 shows an example of application in which the heat exchange (57) between LNG and reactor effluent (15) is done first in a gas/gas heat exchanger, followed by solvent extraction of benzene from the effluent, and second in a LNG vaporizer.

The separation unit is limited by a dotted line box on the flow diagram of Fig. 11. It includes, in addition to the benzene extraction column (59), a compressor (60) and three distillation columns (61), (62), and (63), producing respectively the solvent (C5), hydrogen (11), the recycle stream (13) and the liquid product (24). The actual disposition of the separation unit is not limited to this example nor to the use of distillation columns. Adsorption columns may also be used and their disposition is well known to those skilled in the art. In the example of Fig. 11, the reactor is a cyclone reactor (10) of the type described in Fig. 2 and Fig. 3. The riser (35) and catalyst regeneration (21) by means of a fluidized bed (39) in the regenerator (21) are similar to those of Fig. 4.

In the example of Fig. 11, catalyst transport against gravity from the regenerator (21) to the reactor (10) is by means of a lift column using re-vaporized LNG as lift gas. The fresh catalyst is stored in a hopper (64) while the lift gas is separated in a cyclone (65) recompressed by a compressor (66) and recycled to the reactor inlet. A portion of the separated lift gas (67) may also be used as fuel for the reboilers of the fractionation columns (63), (62), and (61). A furnace (68), preferably electric, heats the lift gas so as to maintain the catalyst temperature at a high level (up to 900°C) prior to its introduction into the reactor (10).

Because this process is simpler than that of Fig. 4, (applicable to natural gas), for LNG a plurality of fixed bed catalytic reactors (69) may be used in lieu of the cyclone reactor and fluidized bed regenerator. This is shown on Fig. 12. One of two reactors (70) is subjected to a regeneration cycle while the other reactors are operated in the conversion mode. During the regeneration cycle, the plasma torch (71) is switched off and the hydrogen flow is interrupted by closing the hydrogen feed valve (72). The residence time in a fixed bed reactor is significantly greater (up to 0.5 sec.) than in a cyclone reactor. To maintain the carbon selectivity at low level, the hydrogen flow is increased (up to 20% of the methane flow), and the spent catalyst temperature is also increased up to 1100°C. Suitable manifolds and valves, not shown on Fig. 12, are used to successively rotate each fixed-bed reactor from the conversion mode to the regeneration mode. This type of operation is well known of those skilled in the art.

Fig. 13 shows an example of application in which the catalytic reactor is of the moving-bed type. A continuous Dow of catalyst, by gravity, is established between the reactor and the regenerator. The fresh catalyst is continuously lifted to the feed hopper (73) by means of a lift pot (74) in which the lift gas is some re-vaporized LNG heated to a temperature of about 600°C.

The present invention is illustrated by the following examples.

Examples:

Example 1

The catalyst is prepared from a solution in ethanol of thorium nitrate, lanthanum nitrate, gallium nitrate, and zirconium nitrate, as described above. Co-precipitation from the solution, buffered with diethanolamine, is achieved with a benzoic acid solution in ethanol. The precipitate, after separation, washing, drying, and calcination, yields a mixed oxide crystal characterized by the following X-ray diffraction spectrum (using a 20 KV source, 1.54059 wave length Cu K alpha-2):

| Angstroms* | Line Intensity (count per second above background) |
|---|---|
| 19.72 | weak (with diffractometer run at 3°/min.) |
| 8.74 | weak |
| 5.05 | very weak |
| 3.74 | weak |
| 3.22 | very strong |
| 2.78 | strong |
| 1.97 | strong |
| 1.69 | strong |
| 1.39 | weak |
| 1.28 | medium |
| 1.13 | medium |
| 1.07 | weak |

*Interplanar spacing

The catalyst is ground in a mortar. Only the fraction comprised between 100 mesh and 230 mesh is retained for the tests. The cyclone reactor configuration shown on Fig. 14 is essentially that of Fig. 8 or that of the reactor in Fig. 11, wherein the cylindrical cavity is made from a fused quartz tube (75) of 3/4" ID (1,90 cm) and 20" (50,8 cm) long oriented vertically. The gaseous effluent from the reactor is carried through a 1/2" (1,27 cm) OD, 3/8" ID (0,95 cm) alumina tube (76), 18" 45,72 cm long, concentric with the quartz tube. A circular groove (77), 1/32" (0.08 cm) deep and 1/4" (0,63 cm) wide is cut by abrasion into the outer surface of the alumina tube (76), 1/2" 1,27 cm from its end. A 3/4" 1,90 cm diameter disk (78) cut from a N°6 gauge platinum foil is cut at six equidistant locations along its periphery-over a 1/8" 0,95 cm radial distance and the six blades thus formed are twisted into helicoidal vanes. Similarly, six radial cuts are made, bisecting the inner part of each helicoidal vane from the center of the disk to a radial distance of 1/4" (0,63 cm). Each of the resulting triangular blades is bent at 90° and the lower part of the alumina tube is pushed into the resulting hexagonal opening, until the bent triangular blades fit snugly into the circular groove of the alumina tube. The shaped platinum piece is held firmly in place by wrapping over the triangular blade six loops of 10 gauge tungsten wire (79). The lower ends of the six wires pass through the slits between the platinum vanes and are gathered below to form a bird-cage configuration pressing against the lower end of the alumina tube. The apex of the bird-cage (80), with all six wires welded together, is centered along the axis of the tube. The upper end of each wire is pulled to about 1" from the other end of the alumina tube and attached to a stainless steel connecting cap (81), closely fitted around the alumina tube (76). The assembly is then inserted into the quartz tube (75), with the connecting cap covering the quartz tube upper end in a gas-tight fit. The connecting cap also includes a tangential pipe connection (82) through which the pre-heated feed gas is injected. The stainless steel piece (81) is electrically grounded. The bottom end of the fused quartz tube (75) is machined by grinding to tightly mate with the conical part of the ceramic shroud (83) of a commercial plasma torch (Secheron Microplasma MP 5-21). The distance between the apex of the tungsten wire bird-cage (80) and the nozzle of the plasma torch is adjusted so that the transferred arc from the torch to the tungsten wires remains stable under normal operating conditions of the microplasma torch (100 to 10,000 Hz pulse rate, hydrogen flow rate 1 to 5 1/m, transferred arc current 0.1 to 5 amp.).

Two short lateral quartz tubes, (84) and (85), 1/8" ID, are fused into the side of the main quartz tube, oriented at an angle of 30° towards each end respectively. These tubes are used for catalyst injection into, and removal from the main quartz tube. Each tube is connected to a stainless steel hopper (86), (87) by means of a block valve (88) in series with a threaded joint (89) and a regulating valve (90). The top branch tube (84) is connected to the bottom of the catalyst feed hopper, and the bottom branch tube to the top of the receiving hopper. The boundaries of the cyclone reactor chamber are respectively at the top of the platinum helicoidal vanes (78), and at the bottom of the ceramic shroud (83) of the microplasma torch. The catalyst is injected just above the platinum vanes and is removed at a level just above the ground joint of the quartz tube.

The effective volume of the reactor chamber thus formed is 11 cc. Electrical heating coils are wrapped respectively around the main quartz tube and around the top catalyst hopper.

The temperature is measured at three points by means of quartz-sheathed Pt-Rh thermocouples:

(a) in the quartz-alumina annular space above the catalyst injection point, (feed gas temperature)

(b) in the reactor chamber at a radial distance of 1/4" from the axis, and 1/2" below the end of the alumina tube, and

(c) within the catalyst feed hopper (catalyst temperature)

Pressure is measured at the inlet gas feed line.

The reactor effluent flowing through the top end of the alumina tube is divided into two streams, the main one exhausting to the atmosphere outside after passing through an oil-filled trap, and the minor one sent to a gas chromatograph for on-line analysis of the conversion products.

The electrical power W applied to the plasma torch, after correction for heat losses to the torch cooling water, is also measured.

Average volume flow rates of feed gas F, hydrogen H, at standard conditions, and the mass flow rate C of catalyst are recorded for each test.

Table 1 summarizes some typical results for pure methane (tests 1, 2, and 3), representative of LNG. These are compared with Test 4 for a C1, C2 mixture representative of the lean gas fraction of a natural gas, as described in Case 1 Process.

## TABLE 1

| Test N° | 1 | 2 | 3 | 4 |
|---|---|---|---|---|
| $T_A$ (°C) | 890 | 890 | 895 | 890 |
| $T_B$ (°C) | 860 | 1050 | 890 | 1050 |
| $T_C$ (°C) | 600 | 600 | NA | 600 |
| P (psia) | 20(1,4 bar) | 20(1,4 bar) | 20(1,4 bar) | 20(1,4 bar) |
| W (watts) | 0 | 340 | 0 | 350 |
| F (1/m) | 30 | 30 | 30 | 29 |
| H (1/m) | 5 | 3 | 3 | 3 |
| C (g/m) | 10 | 10 | 0 | 10 |
| Feed Comp. (volume %) | 100% C1 | 100% C1 | 100%C1 | 93% C1, 7% C2 |

Results    (Average for 10 minute runs)

% weight conversion

| | 5 | 37 | 1 | 45 |
|---|---|---|---|---|

% Liquid selectivity

| | 55 | 64 | 25 | 66 |
|---|---|---|---|---|

Carbon selectivity is very low in all cases.

By-product gas is mostly hydrogen in all cases.

Liquids produced are rich in aromatics.

At the end of each ten minute run, the catalyst feed hopper is almost empty and the receiving hopper is nearly full. All the valves are closed on the two lines connecting the two hoppers to the cyclone reactor, and the threaded joints between each pair of valves are disassembled. The feed hopper is interchanged for the receiving hopper. The threaded joints are re-assembled and thermocouple C is inserted into the hopper located in the heating furnace. The system is ready for another run then the catalyst temperature measured by this thermocouple has stabilized. The four valves on the catalyst flow path are opened and a new run of the cyclone reactor begins.

The conversion of pure methane is also compared with that of propane, representative of the rich gas gas fraction of natural gas as described in the Summary of the Invention. In a separate series of experiments, the bottom line of the feed hopper is disconnected from the cyclone reactor and connected to a propane cylinder. The top outlet of the same hopper is connected to the gas chromatograph and to the gas disposal line. The propane mass flow rate is derived from weight loss of the cylinder. For a catalyst temperature of 600˚C and a propane flow rate of 2 g/m, corresponding to a residence time of six seconds, the propane conversion is 52%, with a liquid selectivity of 71%. At the end of a ten minute conversion run, the propane flow is replaced by a nitrogen flow, followed by an air flow period, resulting in the combustion of the carbon built up. From a measurement of the $CO_2$ produced in the combustion, the carbon built up is estimated at less than 2% of the total carbon in the propane feed during the preceding conversion run.

With the laboratory apparatus shown on Fig. 14, the electrostatic potential of the tungsten wires (79) may also be adjusted to a value different from that of electrical ground. For this purpose, a platinum foil is wrapped around the inner surface of the quartz tube (75), and connected to the electrical ground of the plasma torch. An electrostatic generator (not shown on Fig. 14), also connected to this common ground, maintains the electrostatic potential of the tungsten wires, (79), and of the platinum vanes (78), at a negative value of at least 500 volts. With the plasma torch tungsten tip maintained at a positive voltage, with respect to the common ground, the plasma jet length may be increased. Under these conditions, it is also observed that, at constant electrical power W of the plasma torch, the methane conversion yield is increased. This increase in yield is attributed to the increase in probability of collision between ions generated in the gas phase with adsorbed molecules on the catalyst.

Examples 2-11

In Examples 2-11, the procedure described in Example 1 is followed with the exception that the catalyst prepared from thorium nitrate, lanthanum nitrate, gallium nitrate and zirconium nitrate is replaced by the following starting materials which are mixtures as shown in Table 2 below:

## Table 2

### Catalyst Mixtures[a]

| Ex. | Group III A | Group III B (Lanthanide) | (Actinide) | Group IV B |
|---|---|---|---|---|
| 2 | Gallium | Lanthanum | Thorium | Hafnium |
| 3 | Indium | Cerium | Actinium | Zirconium |
| 4 | Gallium | Cerium | Thorium | Zirconium |
| 5 | Gallium | Europium | Thorium | Zirconium |
| 6 | Indium | Europium | Thorium | Zirconium |
| 7 | Indium | Europium | Thorium | Hafnium |
| 8 | Indium | Lanthanum | Thorium | Zirconium |
| 9 | Indium | Cerium | Thorium | Zirconium |
| 10 | Indium | Lanthanum | Actinium | Zirconium |
| 11 | Tellurium | Lanthanum | Thorium | Zirconium |

[a]These materials originally present as the nitrates as is described in Example 1.

These nitrates are converted to their respective oxides and employed as described in Example 1. These catalysts are expected to produce essentially equivalent hydrogenation results as is described in Example 1.

To summarize, the invention relates to a process for converting all natural gas hydrocarbon components with carbon numbers of 1 to 4 into liquid hydrocarbons with carbon numbers equal to or greater than 5, and into a hydrogen-rich gaseous by-product , said process comprising the following steps :

(a) splitting (1) the natural gas feed into a rich gas stream comprising $C_2$, $C_3$ and $C_4$ hydrocarbons and a lean gas stream comprising $C_1$ and $C_2$ hydrocarbons :

(b) catalytically converting said rich gas stream in a catalytic bed reactor (5) in which the gas-suspended solid phase is a catalyst maintained at a temperature less than 600° C :

(c) separating (7) the gaseous effluent from said catalytic bed reactor into (1) a hydrogen-rich stream (11), (2) a lean gas stream (13) comprising hydrogen, $C_1$ and $C_2$ hydrocarbons, (3) a rich gas stream (4) comprising $C_2$, $C_3$ and $C_4$ hydrocarbons and (4) a liquid product stream comprising $C_5$ + hydrocarbons (24) ;

(d) pre-heating all lean gas streams, including recycle, in a furnace (14) ;

(e) transferring said catalyst into the peripheral inlet of a short residence time reactor (10) (or "cyclone reactor") ;

(f) reacting an ionized plasma derived from said hydrogen stream with said pre-heated lean gas stream carrying said transferred catalyst in said short residence time reactor (10) at a temperature ranging from 600° C to 900° C and at a residence time ranging from 100 to 300 milliseconds, with subsequent quenching of reactions ;

(g) separating the gas-solid stream resulting from said reaction into a spent catalyst phase stream and a gaseous effluent stream through disengagement (17) by centrifugal forces ;

(h) separating said gaseous effluent stream from said disengagement means into four streams as defined in step c, using the same separation facilities (7) as in step c ;

(i) regenerating said spent catalyst in a regenerator (21) by combustion of the carbon build-up on said spent catalyst in an oxidizing gas stream ;

(j) transferring the regenerated catalyst back into said catalytic bed reactor (5) and into said short residence time reactor (10) ;

(k) recycling all rich gas streams obtained in steps c and h back to said catalytic bed reactor (5) ;

(l) recycling the lean gas stream obtained in step h back to said pre-heating furnace (14) of step d.

**Claims**

1. A process for converting all natural gas hydrocarbon components with carbon numbers of 1 to 4 into liquid hydrocarbons with carbon numbers equal to or greater than 5, and into a hydrogen-rich gaseous by-product ; said process comprising the following steps :

(a) splitting (1) the natural gas feed into a rich gas stream comprising $C_2$, $C_3$ and $C_4$ hydrocarbons and a lean gas stream comprising $C_1$ and $C_2$ hydrocarbons ;

(b) catalytically converting said rich gas stream in a catalytic bed reactor (5) in which the gas-suspended solid phase is a catalyst maintained at a temperature less than 600° C ;

(c) separating (7) the gaseous effluent from said catalytic bed reactor into (1) a hydrogen-rich stream (11), (2) a lean gas stream (13) comprising hydrogen, $C_1$ and $C_2$ hydrocarbons, (3) a rich gas stream (4) comprising $C_2$, $C_3$ and $C_4$ hydrocarbons and (4) a liquid product stream comprising $C_5$ + hydrocarbons (24) ;

(d) pre-heating all lean gas streams, including recycle, in a furnace (14) ;

(e) transferring said catalyst into the peripheral inlet of a short residence time reactor (10) (or "cyclone reactor") ;

(f) reacting an ionized plasma derived from said hydrogen stream with said pre-heated lean gas stream carrying said transferred catalyst in said short residence time reactor (10) at a temperature ranging from 600° C to 900° C and at a residence time ranging from 100 to 300 milliseconds, with subsequent quenching of reactions ;

(g) separating the gas-solid stream resulting from said reaction into a spent catalyst phase stream and a gaseous effluent stream through disengagement (17) by centrifugal forces ;

(h) separating said gaseous effluent stream from said disengagement means into four streams as defined in step c, using the same separation facilities (7) as in step c ;

(i) regenerating said spent catalyst in a regenerator (21) by combustion of the carbon build-up on said spent catalyst in an oxidizing gas stream ;

(j) transferring the regenerated catalyst back into said catalytic bed reactor (5) and into said short residence time reactor (10) ;

(k) recycling all rich gas streams obtained in steps c and h back to said catalytic bed reactor (5) ;

(l) recycling the lean gas stream obtained in step h back to said pre-heating furnace (14) of step d.

2. A process according to claim 1 wherein said catalyst used in said catalytic bed and short residence time reactor includes a crystalline form of refractory mixed oxides of composition x $MO_2$, y $M'M''O_3$ and z $M'''O_2$ where x, y and z are mole fractions with values comprised between 0 and 98 %, and where

M       is an Actinide metal,
M'      is a Lanthanide metal,
M''     is gallium,
M'''    is zirconium

and where x is greater than y and y is greater than z.

3. A process according to any one of claims 1 or 2 wherein said crystalline form of refractory mixed oxides is obtained by :

(a) forming a precipitate from a water and alcohol solution of soluble salts of metals M, M', M'', M''' buffered with an organic base, using as a precipitation reagent a solution in water and alcohol of a heat-decomposable acid or a salt of a heat-decomposable acid ;

EP 0 205 598 B1

(b) heating the washed precipitate in an autoclave in the presence of steam ;

(c) air drying the resulting crystals ;

(d) compacting said air dried crystals into pellets ;

(e) heating said pellets in an inert atmosphere exceeding the decomposition temperature of said acid ; and

(f) sintering said pellets at 1000 ° C in the presence of steam.

**Revendications**

1. Procédé de conversion de tous les composants hydrocarbures du gaz naturel ayant de 1 à 4 atomes de carbone en hydrocarbures liquides ayant 5 atomes de carbone ou plus, et en un sous-produit gazeux riche en hydrogène, ledit procédé comportant les étapes suivantes :

(a) séparation (1) de la charge de gaz naturel en un courant de gaz riche comportant des hydrocarbures $C_2$, $C_3$ et $C_4$ et un courant de gaz pauvre comportant des hydrocarbures $C_1$ et $C_2$;

(b) conversion catalytique dudit courant de gaz riche dans un réacteur à lit catalytique (5) dans lequel la phase solide maintenue en suspension par un gaz est un catalyseur dont la température est maintenue inférieure à 600 ° C;

(c) séparation (7) de l'effluent gazeux issu dudit réacteur à lit catalytique en (1) un courant riche en hydrogène (11), (2) un courant de gaz pauvre (13) comportant de l'hydrogène et des hydrocarbures $C_1$ et $C_2$, (3) un courant de gaz riche (4) comportant des hydrocarbures $C_2$, $C_3$ et $C_4$ et (4) un courant de produit liquide comportant des hydrocarbures $C_5^+$ (24);

(d) préchauffage de tous les courants de gaz pauvre, y compris le gaz de recyclage, dans un four (14);

(e) transfert dudit catalyseur dans l'entrée périphérique d'un réacteur à temps de séjour court (10) (ou "réacteur cyclone");

(f) mise en réaction d'un plasma ionisé provenant dudit courant d'hydrogène avec ledit courant de gaz pauvre préchauffé portant ledit catalyseur dans ledit réacteur à temps de séjour court (10) à une température de 600 ° C à 900 ° C et avec un temps de séjour de 100 à 300 millisecondes, et arrêt consécutif des réactions;

(g) séparation du courant gaz-solide issu de ladite réaction en un courant de phase catalytique usée et un courant d'effluent gazeux par séparation (17) au moyen de forces centrifuges;

(h) séparation dudit courant d'effluent gazeux obtenu avec lesdits moyens de séparation, en quatre courants tels que ceux définis à l'étape c, en employant les mêmes moyens de séparation (7) qu'au cours de l'étape c;

(i) régénération dudit catalyseur dans un régénérateur (21) par la combustion du carbone accumulé sur ledit catalyseur usé dans un courant de gaz oxydant;

(j) retour du catalyseur régénéré dans le réacteur à lit catalytique (5) et dans le réacteur à temps de séjour court (10);

(k) recyclage de tous les courants de gaz riche obtenus au cours des étapes c et h vers ledit réacteur à lit catalytique (5);

(l) recyclage du courant de gaz pauvre obtenu à l'étape h vers ledit four de préchauffage (14) de l'étape d.

2. Procédé selon la revendication 1 dans lequel ledit catalyseur utilisé dans ledit réacteur à lit catalytique et à temps de séjour court comporte, sous forme de cristaux, des oxydes mixtes réfractaires de composition x $MO_2$, y $M'M''O_3$ et z $M'''O_2$ où x, y et z sont des fractions moléculaires présentant des valeurs comprises entre 0 et 98 %, et où

M est un métal Actinide,

M' est un métal Lanthanide,

M'' est du gallium,

M''' est du zirconium

et où x est supérieur à y et y est supérieur à z.

3. Procédé selon l'une des revendications 1 ou 2 dans lequel ladite forme cristalline des oxydes mixtes réfractaires est obtenue par :

(a) formation d'un précipité à partir d'une solution d'eau et d'alcool de sels solubles de métaux M, M', M'', M''' tamponnée avec une base organique, en utilisant comme réactif de précipitation une solution dans de l'eau et de l'alcool d'un acide décomposable à la chaleur ou un sel d'acide

15

décomposable à la chaleur;

(b) chauffage du précipité lavé dans un autoclave en présence de vapeur d'eau;

(c) séchage à l'air des cristaux obtenus;

(d) compactage desdits cristaux séchés à l'air sous forme de pastilles;

(e) chauffage desdites pastilles sous atmosphère inerte à une température supérieure à la température de décomposition dudit acide; et

(f) frittage desdites pastilles à 1000° C en présence de vapeur d'eau.

**Patentansprüche**

1. Verfahren zur Umwandlung aller Naturgas-Kohlenwasserstoffkomponenten mit Kohlenstoffzahlen von 1 bis 4 in flüssige Kohlenwasserstoffe mit Kohlenstoffzahlen gleich oder größer als 5, und in ein wasserstoffreiches gasförmiges Nebenprodukt, wobei das Verfahren die folgenden Stufen umfaßt:

(a) Aufspaltung (1) der Naturgasbeschickung in einen reichhaltigen Gasstrom mit $C_2$-, $C_3$- und $C_4$-Kohlenwasserstoffen und einen geringhaltigen Gasstrom mit $C_1$- und $C_2$-Kohlenwasserstoffen;

(b) katalytische Umwandlung des reichhaltigen Gasstroms in einem katalytischen Bettreaktor (5), in dem die gas-suspendierte Feststoffphase ein Katalysator ist, der bei einer Temperatur von weniger als 600° C gehalten wird;

(c) Trennung (7) des gasförmigen Abstroms aus dem katalytischen Bettreaktor in (1) einen wasserstoffreichen Strom (11), (2) einen geringhaltigen Gasstrom (13), der Wasserstoff, $C_1$- und $C_2$-Kohlenwasserstoffe aufweist, (3) einen reichhaltigen Gasstrom (4), der $C_2$-, $C_3$ und $C_4$-Kohlenwasserstoffe aufweist, und (4) einen flüssigen Produktstrom, der $C_{5+}$-Kohlenwasserstoffe (24) aufweist;

(d) Vorerhitzen aller geringhaltigen Gasströme, einschließlich des Recyclisierstroms, in einem Ofen (14);

(e) Überführung des Katalysators in den peripheren Einlaß eines Kurzverweilzeitreaktors (10) (oder "Zyklonreaktors");

(f) Reaktion eines ionisierten Plasmas, gebildet aus dem Wasserstoffstrom mit dem vorerhitzten geringhaltigen Gasstrom, der den überführten Katalysator im Kurzverweilzeitreaktor (10) mit sich führt, bei einer Temperatur, die von 600° C bis 900° C reicht und bei einer Verweilzeit, die von 100 bis 300 Millisekunden reicht, unter nachfolgender Unterdrückung der Reaktionen;

(g) Trennung des aus dieser Reaktion resultierenden Gas-Feststoffstroms in einen Strom aus verbrauchter Katalysatorphase und einen gasförmigen Abflußstrom durch Auftrennung (17) mittels Zentrifugalkräften;

(h) Trennung des gasförmigen Abstroms aus der Auftrenneinheit in vier Ströme wie sie in Stufe c definiert sind, unter Verwendung der gleichen Trennvorrichtung (7), wie in Stufe c;

(i) Regenerierung des verbrauchten Katalysators in einem Regenerator (21) durch Verbrennung der Kohlenstoffablagerung am verbrauchten Katalysator in einem oxidierenden Gasstrom;

(j) Überführung des regenerierten Katalysators zurück in den katalytischen Bettreaktor (5) und in den Kurzverweilzeitreaktor (10);

(k) Recyclisierung aller reichhaltigen Gasströme, die in den Stufen c und h erhalten wurden, zurück in den katalytischen Bettreaktor (5);

(l) Recyclisierung des in Stufe h erhaltenen geringhaltigen Gasstroms zurück in den Vorheizofen (14) der Stufe d.

2. Verfahren nach Anspruch 1, bei dem der in dem katalytischen Bett und dem Kurzverweilzeitreaktor verwendete Katalysator eine kristalline Form von hitzebeständigen Mischoxiden der Zusammensetzung x $MO_2$, y $M'M''O_3$ und z $M'''O_2$ umfaßt, worin x, y und z Molfraktion mit Werten, die zwischen 0 und 98% liegen, bedeuten und worin

M        ein Actinidenmetall ist,

M'       ein Lanthanidenmetall ist,

M''      Gallium ist,

M'''     Zirkonium ist

und worin x größer als y und y größer als z ist.

3. Verfahren nach einem der Ansprüche 1 oder 2 worin die kristalline Form von hitzebeständigen Mischoxiden erhalten wird durch:

(a) Bildung eines Niederschlags aus einer Wasser- und Alkohollösung von löslichen Salzen von Metallen M, M', M'', M''', gepuffert mit einer organischen Base, unter Verwendung einer Lösung in Wasser und Alkohol einer durch Hitze zersetzbaren Säure oder eines Salzes einer durch Hitze zersetzbaren Säure als Ausfällreagens;

(b) Erhitzen des gewaschenen Niederschlags in einem Autoklaven in Gegenwart von Dampf;

(c) Lufttrocknung der erhaltenen Kristalle;

(d) Kompaktierung der luftgetrockneten Kristalle in Pellets;

(e) Erhitzen der Pellets in einer inerten Atmosphäre, die die Zersetzungstemperatur der Säure überschreitet; und

(f) Sintern der Pellets bei 1000 °C in Gegenwart von Dampf.

# fig.1

C₁ C₂ recycle — 13

- 14 Furnace
- 10 cyclone reactor
- 22
- 25
- 16
- 15
- 12
- 11
- 23 fuel
- 3
- disengage-ment
- natural gas feed
- feed splitter unit — 1
- 17  19
- 18
- sepa-ration unit — 7
- C₁ C₂ C₃ C₄
- steam
- catalyst stripper
- 20  21
- 2
- air
- catalyst regenerator
- 9
- 6
- C₅₊ — 24
- 8
- 5 catalytic bed reactor
- 4

C₂ C₃ C₄ recycle

fig.2

fig.3

# fig.4

# fig.5

fig.6

fig.7

fig.8

fig.9

# fig.10

fig.11

EP 0 205 598 B1

# fig.12

28

fig.13

EP 0 205 598 B1

fig.14